# EUROPEAN PATENT APPLICATION

(11) **EP 1 640 355 A1**
(43) Date of publication of application: **29.03.2006**
(21) Application number: 04022718.3
(22) Date of filing: 23.09.2004
(51) Int. Cl.: C07C 17/32

(54) **A practical, cost-effective synthesis of chloromethylated 1,4- benzoquinones**

(71) Applicant: BASF Aktiengesellschaft, 67056 Ludwigshafen (DE)
(72) Inventor: Volker, Berl, Dr., 77694 Kehl (DE); Wetterich, Frank, Dr., 67157 Wachenheim (DE); Hansgeorg, Ernst, Dr., 67346 Speyer (DE); Schein, Karin, Dr., 67065 Ludwigshafen (DE)

(57) **Abstract**

The present invention relates to a practical and cost-effective method for the synthesis of 5-chloromethylated 2,3-dialkoxy-6-alkyl-1,4-benzoquinones by direct chloromethylation of the corresponding 2,3-dialkoxy-6-alkyl-1,4-benzoquinones. The invention further relates to a method for the preparation of 5-chloromethylated 2,3-dialkoxy-6-alkyl-1,4-benzoquinones starting from a 3,4,5-trialkoxy-1-alkyl-benzene.

## Description

The present invention relates to a practical and cost-effective method for the synthesis of 5-chloromethylated 2,3-dialkoxy-6-alkyl-1,4-benzoquinones by direct chloromethylation of the corresponding 2,3-dialkoxy-6-alkyl-1,4-benzoquinones. The invention further relates to a method for the preparation of 5-chloromethylated 2,3-dialkoxy-6-alkyl-1,4-benzoquinones starting from a 3,4,5-trialkoxy-1-alkyl-benzene.

### Background of the Invention

5-Chloromethyl-2,3-dimethoxy-6 methyl-1,4-benzoquinone of formula (I) 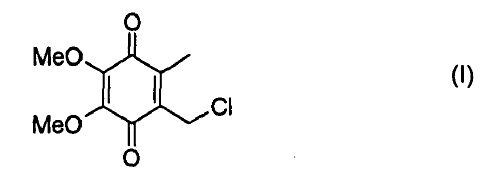 has been shown to be a valuable building block for the synthesis of naturally occurring compounds, especially the ubiquinones, commonly called the coenzymes Qₙ (with n = 1-12). Due to the importance of 5-chloromethyl-2,3-dimethoxy-6 methyl-1,4-benzoquinone as a building block for convergent synthetic strategies towards the ubiquinones a number of protocols have been developed to synthesize chloromethylated quinones.

US-2,998,430 teaches the chloromethylation of alkyl-substituted tocopherol-precursor quinones by treatment with formaldehyde and hydrochloric acid, followed by reduction and cyclization to the corresponding tocopherols.

A. Giraud et al, describe in Tetrahedron Letters, 40 (1999), 4321 -4322 the synthesis of chloromethylated di- and trimethoxy-quinones starting from tetra- or penta-methoxybenzaldehyde derivatives by reduction of the aldehyde functionality, chlorination of the resulting alcohol and finally transformation of aromatic nucleus to the para-quinone by oxidation with cerium-ammonium-nitrate (CAN).

The chloromethylation of 1,4-dimethoxy-2,3-dimethyl-benzene by reaction with formaldehyde in the presence of HCl-gas followed by CAN-oxidation of the resulting chloromethylated aromatic compound has been described by Lipshutz et al in J. Am. Chem. Soc. 121, (1999), 11664 -11673. In the same publication the authors describe the preparation of 5-chloromethyl-2,3-dimethoxy-6-methyl-1,4-benzoquinone by CAN-oxidation of 6-chloromethyl-2,3,4,5-tetramethoxy-toluene. The latter transformation is also described by Lipshutz et al in Tetrahedron, 54 (1998), 1241 -1253.

For the reasons set forth above, a short, practical and cost effective synthesis of 5-chloromethylated 2,3-dialkoxy-6-alkyl-1,4-benzoquinones starting from readily available 2,3-dialkoxy-6-alkyl-1,4-benzoquinones would represent a significant advance in the synthesis of ubiquinones and their analogs.

### Detailed Description of the Invention and Preferred Embodiments

### Definitions

The term "alkyl," by itself or as part of another substituent, means, unless otherwise stated, a straight or branched chain, or cyclic hydrocarbon radical, or combination thereof, which may be fully saturated, mono- or polyunsaturated and can include di- and multi-valent radicals, having the number of carbon atoms designated (i.e. C₁-C₁₀ means one to ten carbons). Examples of saturated hydrocarbon radicals include groups such as methyl, ethyl, n-propyl, isopropyl, n-butyl, t-butyl, isobutyl, sec-butyl, cyclohexyl, (cyclohexyl)ethyl, cyclopropylmethyl, homologs and isomers of, for example, n-pentyl, n-hexyl, n-heptyl, n-octyl, and the like. An unsaturated alkyl group is one having one or more double bonds or triple bonds. Examples of unsaturated alkyl groups include vinyl, 2-propenyl, crotyl, 2-isopentenyl, 2-(butadienyl), 2,4-pentadienyl, 3-(1,4-pentadienyl), ethynyl, 1- and 3-propynyl, 3-butynyl, and the higher homologs and isomers. The term "alkylene" by itself or as part of another substituent means a divalent radical derived from an alkane, as exemplified by-CH₂CH₂CH₂CH₂- . Typically, an alkyl group will have from 1 to 10 carbon atoms, with those groups having 6 or fewer carbon atoms being preferred in the present invention.

The terms "halo" or "halogen," by themselves or as part of another substituent, mean, unless otherwise stated, a fluorine, chlorine, bromine, or iodine atom.

### The Methods

In a first aspect, the invention provides a method for the preparation of tetrasubstituted 1,4-benzoquinones of formula (II) 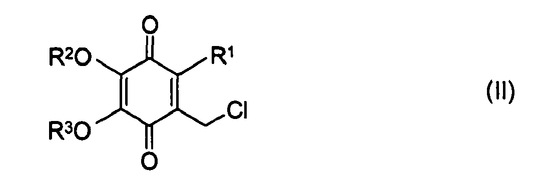 wherein
- R¹, R², R³: are independently selected from the group consisting of branched or unbranched C₁-C₁₀-alkyl, phenyl and benzyl, wherein phenyl and benzyl is optionally substituted by one or more substituents independently selected from the group consisting of C₁-C₆-alkyl and halogen and wherein C₁-C₁₀₋alkyl is optionally substituted by one or more halogen substituents and wherein R₂ and R₃ together can form a C₁-C₆-alkylene radical, optionally substituted by one or more substituents independently selected from the group consisting of C₁-C₆-alkyl, phenyl, benzyl and halogen
by reacting a compound of formula (III) 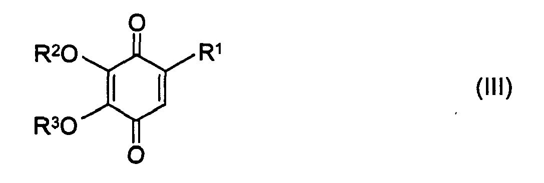 wherein
- R¹, R², R³: are as defined above,
with formaldehyde and/or paraformaldehyde in the presence of hydrochloric acid.

In a preferred embodiment the present invention provides a method for the preparation of tetrasubstituted 1,4-benzoquinones of formula (II), wherein R² and R³ are methyl and R¹ has the meaning as described above for formula (II) starting from a compound of formula (III) wherein R² and R³ are methyl and R¹ is defined as above for formula (II). In a particularly preferred embodiment the invention provides a method for the preparation of tetrasubstituted 1,4-benzoquinones of formula (II), wherein R¹, R² and R³ are methyl starting from a compound of formula (III) wherein R¹, R² and R³ are methyl.

The method according to the present invention for the preparation of chloromethylated 2,3-dialkoxy-6-alkyl-1,4-benzoquinones is characterized in that a 2,3-dialkoxy-6-alkyl-1,4-benzoquinone of formula (I) is reacted with formaldehyde and/or paraformaldehyde in the presence of hydrochloric acid.

The reaction is usually carried out at temperatures from about -20 to about 60°C, preferably at temperatures from about 15 to about 25°C. Usually the reagents are contacted at room temperature in a suitable reaction vessel under appropriate stirring. The reaction can be carried out with or without a solvent or a mixture of solvent. The chemical nature of the solvent is not critical as long as it is inert under the reaction conditions. Suitable solvents are, for example: toluene, benzene, chlorinated solvents such as, for example, dichloromethane, chloroform, tetrachloromethane, tetrachloroethane, chlorobenzene and alkanes such as, for example, hexane, octane or isooctane.

The concentration of the reactants in the solvent or solvent mixture, if used, can be varied over a wide range and is usually chosen between about 0,1 and about 2,0 mol/l with respect to the amount of 2,3-dialkoxy-6-alkyl-1,4-benzoquinone used.

Formaldehyde can be used in any suitable form known to those of skill in the art, for example as a gas, as an aqueous solution (formalin) or in the form of paraformaldehyde. In a preferred embodiment of the present invention formaldehyde is used in the form of paraformaldehyde.

The formaldehyde or paraformaldehyde is usually used in at least equimolar amounts with respect to the 2,3-dialkoxy-6-alkyl-1,4-benzoquinone. Preferably formaldehyde or paraformaldehyde respectively is used in excess, especially in a molar ratio of about 2:1 to 10:1 with respect to the amount of quinone.

The third reagent necessary for the reaction according to the present invention is hydrochloric acid. It can be used in gaseous form or in form of aqueous solution, preferable in form of an aqueous solutions. Preferred aqueous solutions of hydrochloric acid are those with a concentration of about 5 to 36 weight-%. Especially preferred is concentrated hydrochloric acid with a concentration of about 18 to about 36 weight-%.

According to the present invention hydrochloric acid is usually used in at least equimolar amounts with respect to the 2,3-dialkoxy-6-alkyl-1,4-benzoquinone. Preferably hydrochloric acid is used in excess amounts, especially in a molar ratio of about 2:1 to about 20:1 with respect to the amount of quinone. In a preferred embodiment hydrochloric acid is used in molar excess compared to formaldehyde. Best results are usually obtained when at least 1,05 to about 4, preferably about 1,5 to about 3 equivalents of hydrochloric acid are used with respect to the amount of formaldehyde used.

As mentioned earlier the three reactants are brought in contact and mixed, preferably by stirring. Depending on the exact reaction conditions the reaction is usually complete after about 10 minutes to 10 h, oftentimes after about 15 Minutes to about 3 h.

In a particularly preferred embodiment of the present invention a 2,3-dialkoxy-6-alkyl-1,4-benzoquinone, preferably 2,3-dimethoxy-6-methyl-1,4-benzoquinone and paraformaldehyde is dissolved in toluene and concentrated hydrochloric acid is added dropwise over a period of about 30 min to about 1 h to the stirred mixture at temperatures from 15 to 25°C. After complete addition the reaction mixture is usually stirred for further 2 to 3 h at that temperature.

The reaction mixture obtained can be worked up by all techniques known to those of skill in the art. Usually the solids formed during the reaction are filtered off and the organic phase is separated and dried to afford a crude product which can be separated or purified by all suitable, art-recognized techniques, especially by cristallization, chromotography or distillation, preferably under reduced pressure and temperatures below 140°C, especially by short-path distillation.

In another aspect, the present invention provides a short, economic, two-step method for the preparation of 5-chloromethylated 2,3-dialkoxy-6-alkyl-1,4-benzoquinones starting from readily available 3,4,5-trialkoxy-1-alkyl-benzenes of formula (IV). According to this aspect of the invention a compound of formula (III) is prepared by oxidation of a compound of formula (IV) 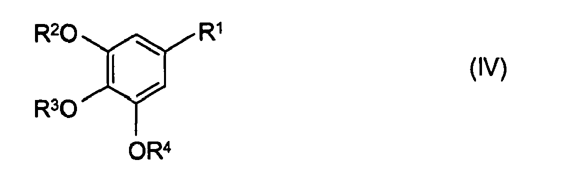 wherein
- R¹, R², R³, R⁴: are independently selected from the group consisting of branched or unbranched C₁-C₁₀-alkyl, phenyl and benzyl, wherein phenyl and benzyl is optionally substituted by one or more substituents independently selected from the group consisting of C₁-C₆-alkyl and halogen and wherein C₁-C₁₀-alkyl is optionally substituted by one or more halogen substituents and wherein R₂ and R₃ together can form a C₁₋C₆-alkylene radical, optionally substituted by one or more substituents independently selected from the group consisting of C₁-C₆-alkyl, phenyl, benzyl and halogen.

The compound of formula (III) prepared according to this aspect of the present invention is then used for the preparation of a compound of formula (II) according to the method described above.

According to this aspect the present invention provides a method for the preparation of compounds of formula (II) comprising
a) the oxidation of a compound of formula (IV) wherein R¹, R², R³, R⁴ are as defined above to a compound of formula (III) wherein R¹, R², R³ are as defined above and
b) reacting the compound of formula (III) prepared that way with formaldehyde and/or paraformaldehyde in the presence of hydrochloric acid.

The preparation of 2,3-dialkoxy-2-alkyl-1,4-benzoquinones by oxidation of the corresponding aromatic precursors is generally known and described for a large variety of oxidizing agents, for example in JP-A 02138146, EP-A 347 021, DE-A 19736428 or JP-A 07010800.

Suitable reagents for the oxidation of 3,4,5-trialkoxy-1-alkyl-benzenes of formula (IV) to 2,3-dialkoxy-2-alkyl-1,4-benzoquinones of formula (III) are for example: hydrogen peroxide, especially in the presence of suitable acids, for example formic acid or acetic acid, or catalysts, performic acid, peracetic acid, perpropionic acid , (NH₄)₂S₂O₈ and others.

In combination with the method for the chloromethylation of 2,3-dialkoxy-2-alkyl-1,4-benzoquinones according to the present invention this method provides the shortest and most economic access to 5-chloromethyl-2,3-dialkoxy-6-alkyl-1,4-benzoquinones, especially to 5-chloromethyl-2,3-dimethoxy-6-methyl-1,4-benzoquinone known.

The method according to this aspect of the present invention is especially suitable for the preparation of a compound of formula (III), wherein R² and R³ are methyl by oxidation of a compound of formula (IVa) 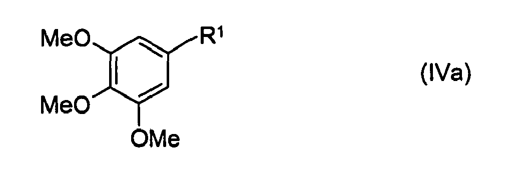 wherein R¹ is defined as above for formula (IV) and chloromethylating the resulting 1,4-benzoquinone as described above.

In a particularly preferred embodiment the method according to this aspect of the present invention is suitable for the preparation of 5-chloromethyl-2,3-dimethoxy-6-methyl-1,4-benzoquinone of formula (I) by oxidation of 3,4,5-trimethoxy-toluene of formula (IVb) 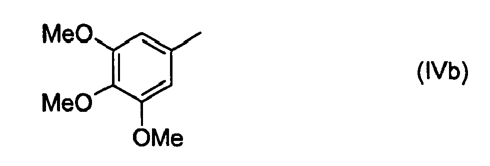 to 2,3-dimethoxy-6-methyl-1,4-benzoquinone followed by chloromethylation under the conditions as described above.

According to the present invention the compounds of formula (III) prepared by oxidation of the compounds of formulae (IV), (IVa) or (IVb) respectively are then chloromethylated as described above.

The compounds prepared according to the present invention are valuable intermediates for the synthesis of a variety of ubiquinones, for example coenzyme Q₁₀ or derivatives or analogues thereof.

A further aspect the present invention therefore relates to the use of the compounds of formula (II) for the preparation of ubiquinones or derivatives or analogues thereof, especiallly coenzyme Q₁₀ or derivatives or analogues thereof.

### Example

The following example provides a representative experimental procedure which is offered to illustrate the present invention without limiting it in any sense:

A reactor with a volume of 6 l equipped with a mechanical stirrer was filled with 2,3-dimethoxy-5-methylbenzoquinone (128 g, 0.70 mol), parafomaldehyde (54.2 g, 2.7 mol (formaldehyde equivalents), and 5 l of toluene. Concentrated Hydrochloric acid (422 g, 4.2 mol HCl) was added to the mixture slowly over a period of 30 minutes via a dropping funnel at room temperature. After completion of the addition the mixture was allowed to stir for another 2.5 h at room temperature, upon which time the remaining solids in the reaction mixture were filtered over a frit, the water phase separated, the toluene phase dried over MgSO₄ and evaporated.

The crude mixture contained the desired 2,3-dimethoxy-5-methylbenzoquinone as the main compound (ca. 60%), and was purified via column chromatography over silica, using a 3:1 mixture of heptane and ethyl acetate.

## Claims

1. A method for the preparation of tetrasubstituted 1,4-benzoquinones of formula (II) 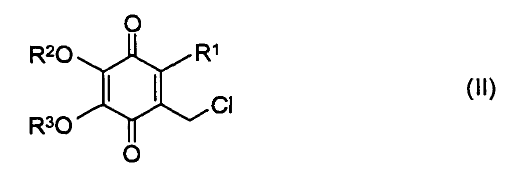 wherein
R¹, R², R³ are independently selected from the group consisting of branched or unbranched C₁-C₁₀-alkyl, phenyl and benzyl, wherein phenyl and benzyl is optionally substituted by one or more substituents independently selected from the group consisting of C₁-C₆-alkyl and halogen and wherein C₁-C₁₀-alkyl is optionally substituted by one or more halogen substituents and wherein R₂ and R₃ together can form a C₁-C₆-alkylene radical, optionally substituted by one or more substituents independently selected from the group consisting of C₁-C₆-alkyl, phenyl, benzyl and halogen
by reaction of a compound of formula (III) 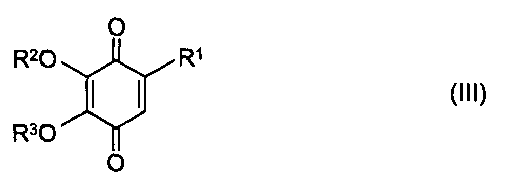 wherein
R¹, R², R³ are as defined above,
with formaldehyde and/or paraformaldehyde in the presence of hydrochloric acid.

2. The method according to claim 1, wherein R² and R³ are methyl.

3. The method according to claim 2, wherein R¹ is methyl.

4. The method according to any one of claims 1 to 3, wherein said reaction is carried out at temperatures from 15 to 25°C.

5. The method according to any one of claims 1 to 4, wherein the reaction is carried out with paraformaldehyde.

6. The method according to any one of claims 1 to 5, wherein the molar ratio between formaldehyde or paraformaldehyde and the compound of formula (III) is 2:1 to 10:1.

7. The method according to any one of claims 1 to 6, wherein the molar ratio between hydrochloric acid and formaldehyde or paraformaldehyde is 1,5:1 to 3:1.

8. The method according to any one of claims 1 to 7, wherein the compound of formula (III) is prepared by oxidation of a compound of formula (IV) 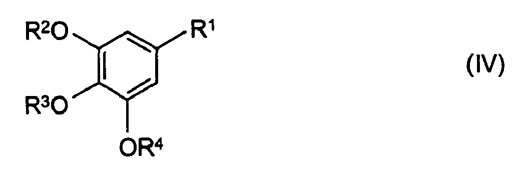 wherein
R¹, R², R³, R⁴ are independently selected from the group consisting of branched or unbranched C₁-C₁₀-alkyl, phenyl and benzyl, wherein phenyl and benzyl is optionally substituted by one or more substituents independently selected from the group consisting of C₁-C₆-alkyl and halogen and wherein C₁-C₁₀-alkyl is optionally substituted by one or more halogen substituents and wherein R₂ and R₃ together can form a C₁-C₆-alkylene radical, optionally substituted by one or more substituents independently selected from the group consisting of C₁-C₆-alkyl, phenyl, benzyl and halogen.

9. The method according to claim 8, wherein the compound of formula (III) is prepared by oxidation of a compound of formula (IVa) 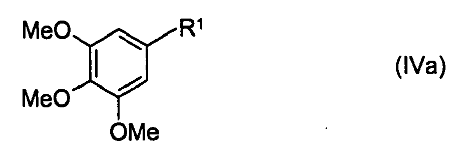 wherein R¹ is defined as above for formula (IV).

10. The method according to claim 8, wherein the compound of formula (III) is prepared by oxidation of 3,4,5-trimethoxy-toluene of formula (IVb)
